# EUROPEAN PATENT APPLICATION

(11) **EP 4 169 932 A1**
(43) Date of publication of application: **26.04.2023**
(21) Application number: 21827869.5
(22) Date of filing: 23.06.2021
(51) Int. Cl.: C07K 1/00, A23J 3/04, A23J 3/14, A23L 33/18

(54) **METHOD FOR CLEAVING DISULFIDE BOND IN PROTEIN AND DEVICE FOR CLEAVING DISULFIDE BOND IN PROTEIN**

(30) Priority: 23.06.2020 JP 2020108085
(71) Applicant: Panasonic Intellectual Property Management Co., Ltd., Osaka-shi, Osaka 540-6207 (JP)
(72) Inventor: HATSUGAI, Noriyuki, Osaka-shi, Osaka 540-6207 (JP); OKUMURA, Yasuaki, Osaka-shi, Osaka 540-6207 (JP); WAYAMA, Fumiya, Osaka-shi, Osaka 540-6207 (JP)
(74) Representative: Novagraaf International SA
(86) International application number: PCT/JP2021/023750
(87) International publication number: WO 2021/261509

(57) **Abstract**

A method for cleaving a disulfide bond in a protein includes: cleaving a disulfide bond in a protein present in a reaction system by reduction via a reduced redox protein (step S104); and reducing an oxidized redox protein produced by oxidation of the reduced redox protein in the cleaving to the reduced redox protein by donating an electron from an electrode connected to an external power supply outside the reaction system to the oxidized redox protein (step S103).

## Description

### [Technical Field]

The present disclosure relates to a method for cleaving disulfide bonds in proteins and a device for cleaving disulfide bonds in proteins.

### [Background Art]

Digestion-resistant proteins are found, for example, in plant-based foods as well as animal-based foods. For example, Patent Literature (PTL) 1 discloses that alkaline treatment of grains such as brown rice improves enzymatic digestion of digestion-resistant proteins by digestive enzymes.

For example, PTL 2 discloses that adding thioredoxin, which is a low molecule weight redox protein, to food to cleave the disulfide bonds of the digestion-resistant proteins in the food improves digestion of the digestion-resistant proteins by digestive enzymes.

### [Citation List]

### [Patent Literature]

[PTL 1] Japanese Unexamined Patent Application Publication No. H03-228669
[PTL 2] Japanese Unexamined Patent Application Publication (Translation of PCT Application) No. 2001-520027

### [Summary of Invention]

### [Technical Problem]

Unfortunately, in the conventional technique described in PTL 1, when digestion-resistant proteins undergo alkaline denaturation, the digestibility of digestion-resistant proteins by enzymes improves, but more (i.e., an excess amount of) enzymes must be added to the food than is required to digest the digestion-resistant proteins.

The conventional technique described in PTL 2 requires adding an excess amount of thioredoxin (i.e., low molecular weight redox protein) relative to the amount of digestion-resistant proteins in order to cleave the disulfide bonds of all digestion-resistant proteins in the food.

These conventional techniques therefore cannot efficiently reduce and cleave disulfide bonds in proteins such as digestion-resistant proteins. Consequently, these conventional techniques can hardly be said to efficiently digest digestion-resistant proteins. Furthermore, with these conventional techniques, a large amount of unreacted enzyme remains in the food.

In view of this, the present disclosure provides a method for cleaving disulfide bonds in proteins and a device for cleaving disulfide bonds in proteins that can repeatedly activate enzymes (hereinafter also referred to as redox proteins) to efficiently reduce and cleave the disulfide bonds in the proteins with a small amount of enzyme (i.e., redox protein).

### [Solution to Problem]

A method for cleaving a disulfide bond in a protein according to one aspect of the present disclosure includes: cleaving a disulfide bond in a protein present in a reaction system by reduction via a reduced redox protein; and reducing an oxidized redox protein produced by oxidation of the reduced redox protein in the cleaving to the reduced redox protein by donating an electron from an electrode connected to an external power supply outside the reaction system to the oxidized redox protein.

A device for cleaving a disulfide bond in a protein according to one aspect of the present disclosure includes: an electrode for donating an electron to a redox protein that cleaves a disulfide bond in a protein by reduction by application of voltage; a power supply that applies voltage to the electrode; and a controller that controls application of voltage by the power supply.

### [Advantageous Effects of Invention]

The present disclosure can provide a method for cleaving disulfide bonds in proteins and a device for cleaving disulfide bonds in proteins that can efficiently reduce and cleave disulfide bonds in proteins with a small amount of redox protein by repeatedly activating the redox proteins.

### [Brief Description of Drawings]

[FIG. 1]

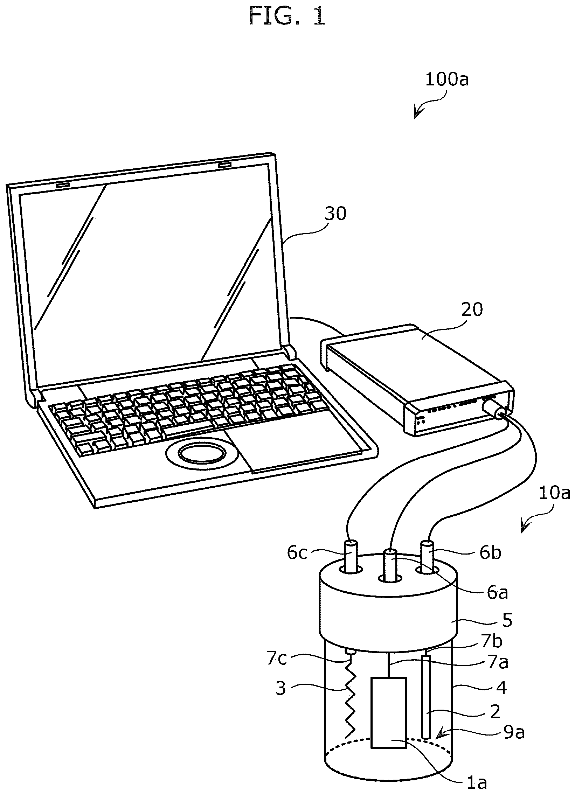

   FIG. 1 illustrates one example of the configuration of a device for cleaving disulfide bonds in proteins according to Embodiment 1.
[FIG. 2]

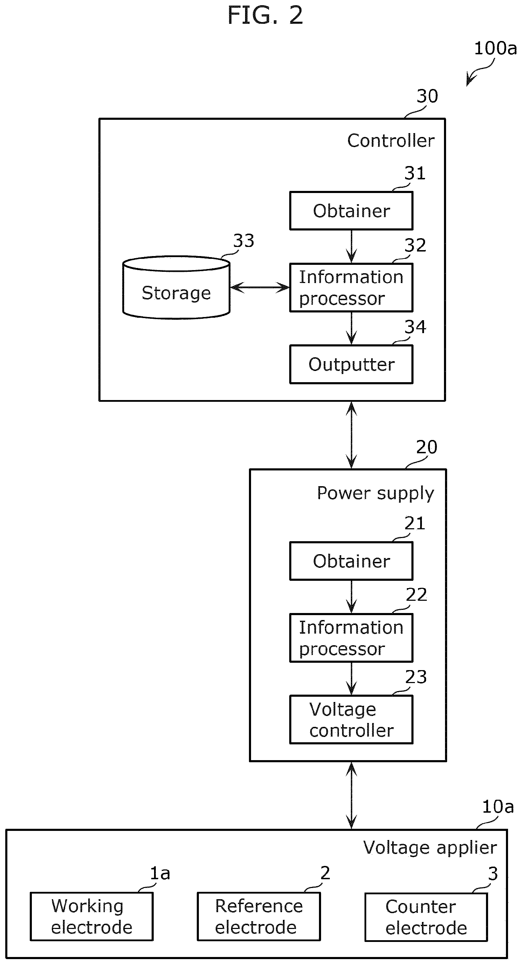

   FIG. 2 is a block diagram illustrating one example of the functional configuration of the device for cleaving disulfide bonds in proteins according to Embodiment 1.
[FIG. 3]

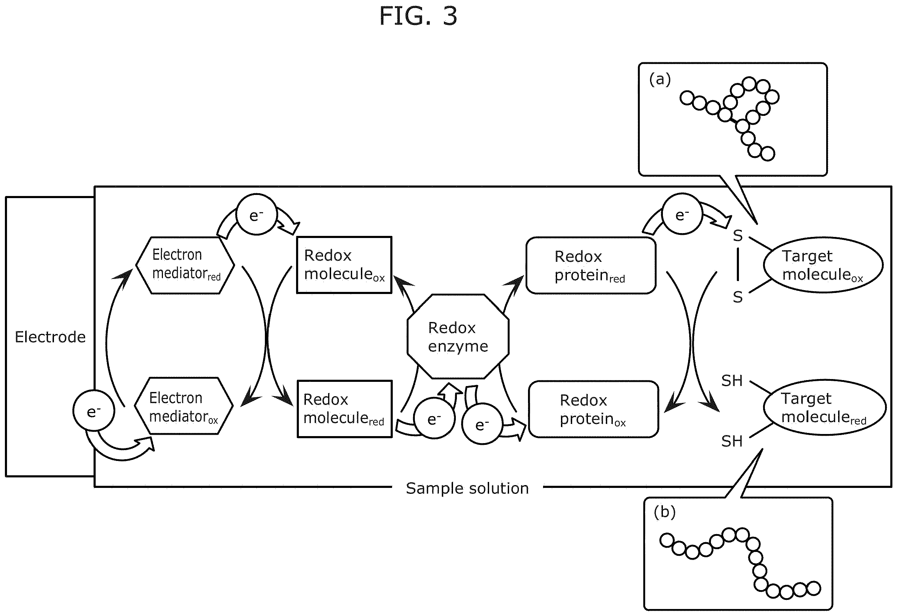

   FIG. 3 is a schematic diagram illustrating the components in a sample solution and the electron transfer reactions between them.
[FIG. 4]

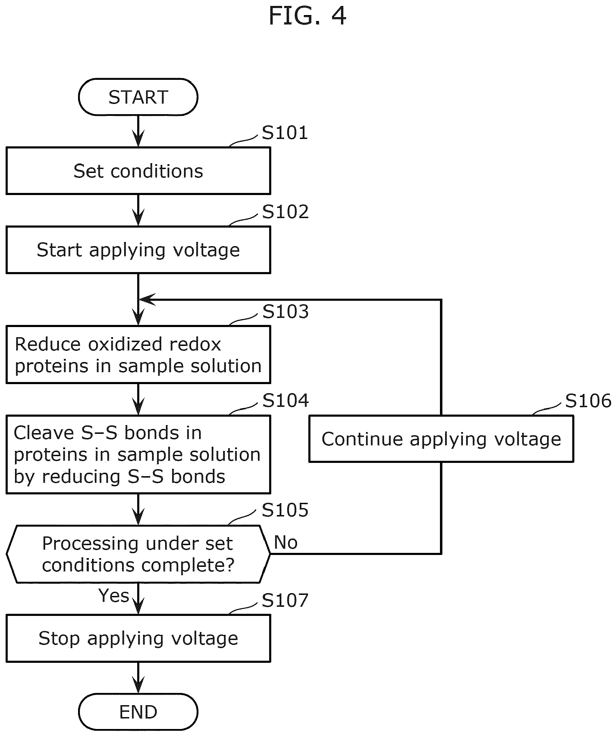

   FIG. 4 is a flowchart illustrating one example of the operation of the device for cleaving disulfide bonds in proteins according to Embodiment 1.
[FIG. 5]

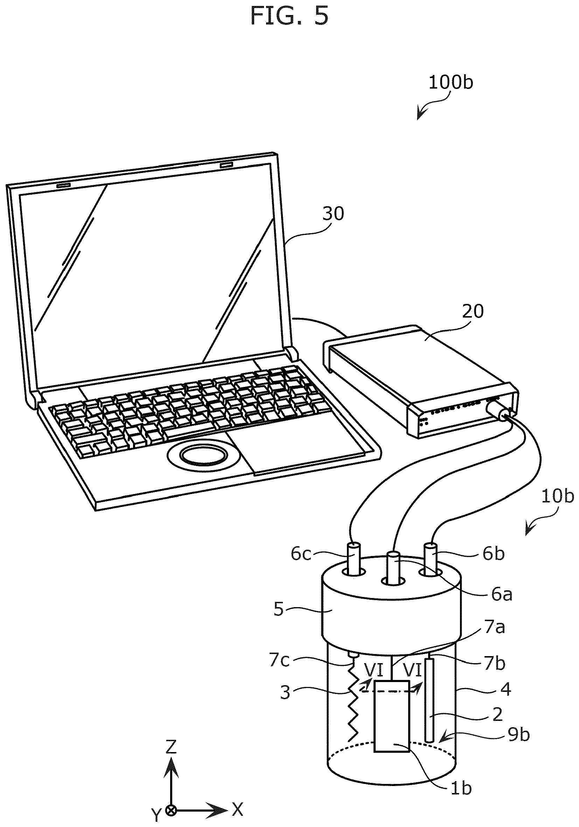

   FIG. 5 illustrates one example of the configuration of a device for cleaving disulfide bonds in proteins according to Embodiment 2.
[FIG. 6]

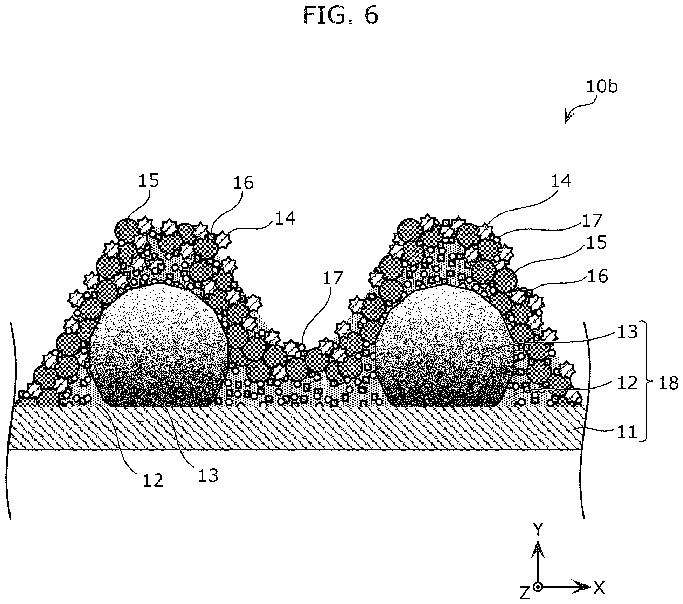

   FIG. 6 is a schematic cross-sectional view taken at line VI-VI of the working electrode illustrated in FIG. 5.
[FIG. 7]

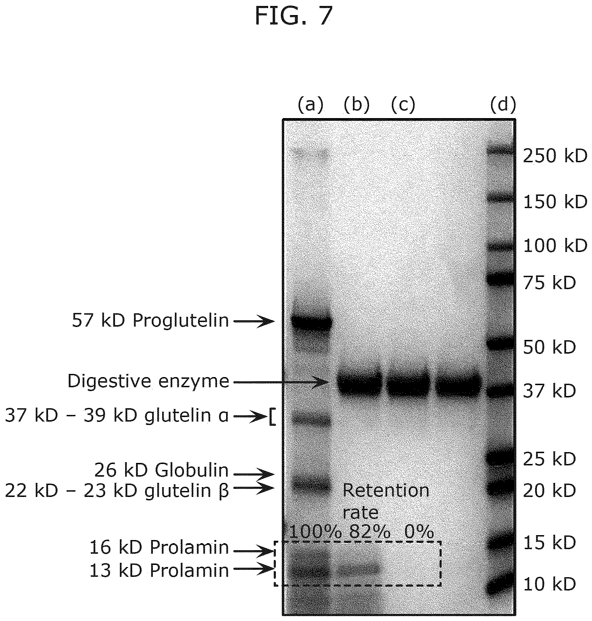

   FIG. 7 illustrates electrophoresis images after SDS-PAGE in Comparative Example 1, Comparative Example 2, and Implementation Example 1.
[FIG. 8]

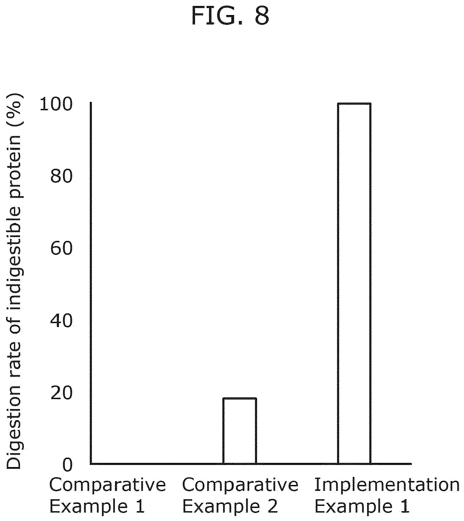

   FIG. 8 is a graph illustrating the digestion rate of prolamin after treatment with digestive enzymes in Comparative Example 1, Comparative Example 2, and Implementation Example 1.
[FIG. 9]

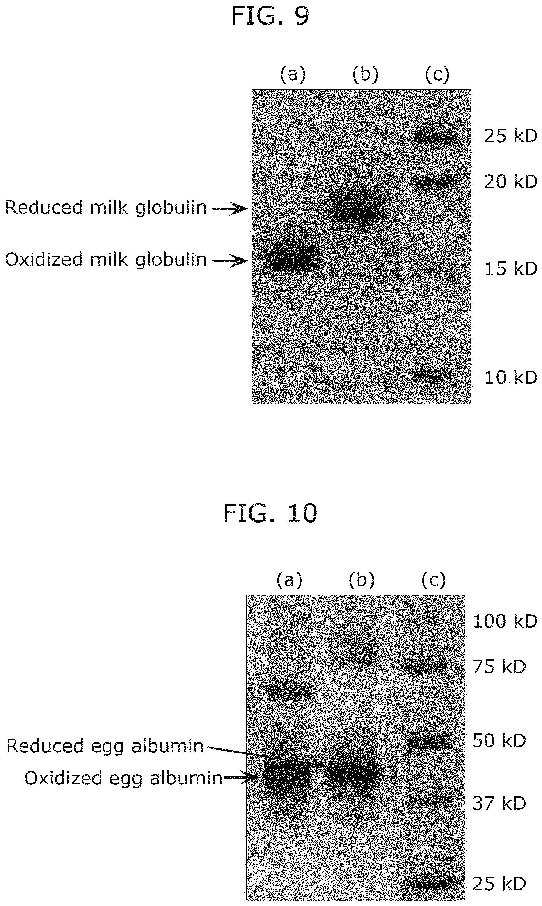

   FIG. 9 illustrates electrophoresis images after SDS-PAGE in Comparative Example 3 and Implementation Example 2.
[FIG. 10]
   FIG. 10 illustrates electrophoresis images after SDS-PAGE in Comparative Example 4 and Implementation Example 3.

### [Description of Embodiments]

### Knowledge Leading to Present Disclosure

In recent years, with changes in dietary habits and the aging of the population, techniques have been developed to improve the digestibility of digestion-resistant substances in foods. Digestion-resistant substances are those that are difficult to digest in the human digestive tract, one example of which being digestion-resistant proteins. Digestion-resistant proteins are found in plant-based foods such as grains and legumes, as well as animal-based foods.

Techniques to increase the digestibility of digestion-resistant substances in foods include, for example, adding heat, adding water, alkali treatment, acid treatment, enzymatic treatment, and combinations thereof. For example, PTL 1 discloses that alkali treatment of grains such as brown rice followed by treatment with proteolytic enzymes improves the digestibility of digestion-resistant proteins in brown rice. However, with the technique described in PTL 1, alkali penetrates into the interior of the food during alkali treatment, so alkali may remain in the food even after acid treatment to neutralize the alkali. In addition, the technique described in PTL 1 adds more enzyme than is necessary to digest the amount of digestion-resistant protein in the food (i.e., an excess amount), so it is difficult to say that the digestion-resistant proteins are being digested efficiently. Furthermore, a large amount of unreacted enzyme remains in the food after enzymatic treatment.

Typically, enzymatic treatment uses an excess amount of enzyme relative to the amount of substrate (for example, digestion-resistant protein). Enzymes are catalytic proteins, which catalyze most chemical reactions in living organisms (for example, redox reactions, hydrolysis reactions, isomerization reactions, elimination addition reactions, or synthesis reactions). Stated differently, enzymes have the ability to lower the activation energy required for a chemical reaction, increasing the rate of the reaction without itself changing (in other words, without the enzyme itself degrading or synthesized into another substance). The enzyme has a binding domain, which is a site that binds to a specific substrate, and a catalytic domain, which acts as a catalyst. These domains are located in close proximity (also called the active center or active site of the enzyme) in the conformational structure of the enzyme (also called the enzyme protein). These domains allow the enzyme to react specifically with the substrate (hereafter referred to as target molecule). Stated differently, enzymes have a property called substrate specificity, which means that if the shape of the reaction site of the substrate and the enzyme do not match, they will not react. Some enzymes are unable to react with substrates on their own and require an organic atom group other than protein, called a coenzyme, to react with the substrate. In this case, the coenzyme binds to the enzyme, allowing it to react specifically with the substrate. Thus, the substrate specificity of the enzyme allows it to efficiently obtain the desired product. In nature, such enzymatic reactions occur repeatedly. For example, in biological reactions in plants, deactivated substances (for example, enzymes, coenzymes, and substances involved in their electron transfer) can be repeatedly activated by energy obtained through photosynthesis. In in vitro reactions, however, once an enzyme loses energy (i.e., is inactivated) by reacting with a substrate, it does not regain the lost energy (in other words, it is not reactivated). One known method of activating inactivated enzymes is to add enzymes that reduce and activate inactivated enzymes (i.e., reductases) and coenzymes that activate reductases (hereinafter referred to as reducing molecules) to the reaction system together with the enzymes. However, in ex vivo reactions, the energy to activate reductases and reducing molecules is not provided to the reaction system, so enzymes are only activated a number of times dependent on the amount of reductase and reducing molecules.

In view of the above, as a result of diligent work, the inventors of the present application have discovered a method of continuously activating enzymes by combining electrochemical techniques with in vivo reactions (i.e., enzymatic reactions). More specifically, the inventors have discovered that by applying voltage to the electrodes, electrical energy can be applied to the reaction system as a replacement for light energy to achieve a repetitive, almost semi-permanent reaction that repeatedly activates inactivated enzymes. The inventors discovered that this allows for processing with a small amount of enzyme relative to the amount of protein (i.e., the number of disulfide bonds in the proteins).

Accordingly, the present disclosure provides a method for cleaving disulfide bonds in proteins and a device for cleaving disulfide bonds in proteins that can efficiently cleave disulfide bonds in proteins since they can efficiently reduce the disulfide bonds in the proteins with a small amount of enzyme (i.e., redox protein) by repeatedly activating the enzymes (i.e., redox proteins).

In the following, "enzymes" will be referred to as "redox proteins," "reductases" that reduce enzymes will be referred to as "redox enzymes," "coenzymes" and molecules that act like coenzymes will be referred to as "redox molecules," and substances involved in electron transfer will be referred to as "electron mediators".

### One Aspect of the Present Disclosure

Hereinafter, an overview of one aspect of the present disclosure will be given.

A method for cleaving a disulfide bond in a protein according to one aspect of the present disclosure includes: cleaving a disulfide bond in a protein present in a reaction system by reduction via a reduced redox protein; and reducing an oxidized redox protein produced by oxidation of the reduced redox protein in the digestion improving to the reduced redox protein by donating an electron from an electrode connected to an external power supply outside the reaction system to the oxidized redox protein.

With this, since oxidized redox proteins can be reduced to reduced redox proteins, redox proteins that once lost their activity can be reactivated and reused to reduce disulfide bonds in proteins. Therefore, a small amount of redox protein relative to the amount of protein can be used to reduce the disulfide bonds in the proteins in the reaction system. Therefore, according to the method for cleaving disulfide bonds in proteins, since disulfide bonds in proteins can be efficiently reduced with a small amount of redox protein, the disulfide bonds in the proteins can be efficiently cleaved.

In the method for cleaving a disulfide bond in a protein according to one aspect of the present disclosure, in the reducing, an electron may be donated from the electrode to a redox enzyme, and an electron may be donated from the redox enzyme to the oxidized redox protein.

With this, the transfer rate and the amount of energy of electrons donated from the electrode to the oxidized redox proteins can be adjusted depending on the combination of the redox enzyme and the oxidized redox protein used. The method for cleaving disulfide bonds in proteins can therefore improve the efficiency of the electron transfer reaction between the electrode and the redox proteins.

In the method for cleaving a disulfide bond in a protein according to one aspect of the present disclosure, in the reducing, an electron may be donated from the electrode to a redox molecule, an electron may be donated from the redox molecule to a redox enzyme, and an electron may be donated from the redox enzyme to the oxidized redox protein.

With this, the transfer rate and the amount of energy of electrons donated from the electrode to the oxidized redox proteins can be adjusted depending on the combination of the redox molecule, the redox enzyme, and the oxidized redox protein used. The method for cleaving disulfide bonds in proteins can therefore improve the efficiency of the electron transfer reaction between the electrode and the redox proteins.

In the method for cleaving a disulfide bond in a protein according to one aspect of the present disclosure, in the reducing, an electron may be donated from the electrode to an electron mediator, an electron may be donated from the electrode mediator to a redox molecule, an electron may be donated from the redox molecule to a redox enzyme, and an electron may be donated from the redox enzyme to the oxidized redox protein.

With this, the transfer rate and the amount of energy of electrons donated from the electrode to the oxidized redox proteins can be adjusted depending on the combination of the electron mediator, the redox molecule, the redox enzyme, and the oxidized redox protein used. The method for cleaving disulfide bonds in proteins can therefore improve the efficiency of the electron transfer reaction between the electrode and the redox proteins.

In the method for cleaving a disulfide bond in a protein according to one aspect of the present disclosure, the protein present in the reaction system may be a digestion-resistant protein. For example, the digestion-resistant protein may be at least one of prolamin, egg albumin, or β-lactoglobulin.

With this, since the disulfide bonds in the digestion-resistant proteins are reduced to thiol groups by the reduced redox proteins, the disulfide bonds in these proteins are cleaved. The connection between secondary structures of the protein immobilized by the disulfide bond is broken, and the degree of freedom (fluctuation) of the protein's conformational structure increases. As a result, it easier for digestive enzymes to act on the cleavage sites where the peptide chain of the digestion-resistant protein is cleaved by the digestive enzymes. Therefore, according to the method for cleaving disulfide bonds in proteins, the digestion of digestion-resistant proteins can be improved because the cleavage of disulfide bonds in digestion-resistant proteins makes it easier for digestive enzymes to act on the cleavage sites in these proteins.

In the method for cleaving a disulfide bond in a protein according to one aspect of the present disclosure, the redox protein may be thioredoxin, glutathione, a protein with at least one thioredoxin-like domain, or a protein with at least one glutathione-like motif.

With this, the redox protein can reduce disulfide bonds because it includes a cysteine-derived thiol group. Therefore, according to the method for cleaving disulfide bonds in proteins, disulfide bonds in proteins can be cleaved by reduction.

In the method for cleaving a disulfide bond in a protein according to one aspect of the present disclosure, the redox enzyme may be: (i) an enzyme that catalyzes reduction of oxidized thioredoxin, including a NADPH-thioredoxin reductase or a ferredoxin-thioredoxin reductase; or (ii) an enzyme that catalyzes reduction of oxidized glutathione, including a glutathione reductase.

With this, when the redox protein is thioredoxin or glutathione, the redox enzymes can efficiently reduce the oxidized redox proteins produced by reducing the disulfide bonds to the reduced redox proteins. Therefore, according to the method for cleaving disulfide bonds in proteins, since disulfide bonds in proteins can be efficiently reduced with a small amount of redox protein, the disulfide bonds in the proteins can be efficiently cleaved.

In the method for cleaving a disulfide bond in a protein according to one aspect of the present disclosure, the redox molecule may be nicotinamide adenine dinucleotide phosphate or ferredoxin.

With this, the redox molecules can donate electrons to the redox enzymes efficiently, thus efficiently reducing the redox enzymes. The redox enzymes can therefore efficiently reduce oxidized redox proteins. Therefore, according to the method for cleaving disulfide bonds in proteins, since disulfide bonds in proteins can be efficiently reduced with a small amount of redox protein, the disulfide bonds in the proteins can be efficiently cleaved.

In the method for cleaving a disulfide bond in a protein according to one aspect of the present disclosure, the electron mediator may be a compound with a bipyridine skeleton.

With this, since the electron mediator has multiple nitrogen-containing heterocyclic rings, the electrons necessary for the redox protein reduction are efficiently donated to the redox molecule due to the multiple contributions of the nitrogen contained in the nitrogen-containing heterocyclic rings. The method for cleaving disulfide bonds in proteins can therefore efficiently donate electrons donated from the electrode via the electron mediators to the oxidized redox proteins. With this, since oxidized redox protein can be efficiently activated, the digestion-resistant proteins can be efficiently digested with a small amount of redox protein. Therefore, according to the method for cleaving disulfide bonds in proteins, since disulfide bonds in proteins can be efficiently reduced with a small amount of redox protein, the disulfide bonds in the proteins can be efficiently cleaved.

In the method for cleaving a disulfide bond in a protein according to one aspect of the present disclosure, a reaction temperature in the reaction system may be greater than or equal to 4 °C and less than 60 °C.

With this, the reduced redox proteins can reduce disulfide bonds in proteins in an environment greater than or equal to 4 °C and less than 60 °C. Therefore, according to the method for cleaving disulfide bonds in proteins, disulfide bonds in proteins can be cleaved in an environment greater than or equal to 4 °C and less than 60 °C.

In the method for cleaving a disulfide bond in a protein according to one aspect of the present disclosure, a voltage applied to the electrode by the external power supply may be between -1.0 V and 0 V, inclusive.

With this, since the applied voltage is adjusted so that the voltage applied to the electrode by the external power supply is within the range of -0.1 V to 0 V, inclusive, the method for cleaving disulfide bonds in proteins can adjust the efficiency of the electron transfer reaction according to the components in the reaction system.

A device for cleaving a disulfide bond in a protein according to one aspect of the present disclosure includes: an electrode for donating an electron to a redox protein that cleaves a disulfide bond in a protein by reduction by application of voltage; a power supply that applies voltage to the electrode; and a controller that controls application of voltage by the power supply.

With this, the device for cleaving disulfide bonds in proteins can donate electrons from the electrode to redox proteins in oxidized form (i.e., oxidized redox proteins), which are produced by oxidizing the disulfide bonds in proteins by reduction, to reduce them to redox proteins in reduced form (i.e., reduced redox proteins). The device for cleaving disulfide bonds in proteins can therefore activate redox proteins that once lost their activity due to redox reactions with disulfide bonds in proteins, and reuse them to reduce disulfide bonds in proteins. Therefore, since the device for cleaving disulfide bonds in proteins can efficiently reduce disulfide bonds in proteins with a small amount of redox protein, the device can efficiently cleave the disulfide bonds in the proteins.

In the device for cleaving a disulfide bond in a protein according to one aspect of the present disclosure, for example, the redox protein may be immobilized on the electrode.

This eliminates the need to add redox proteins to the protein-containing sample. Therefore, since the device for cleaving disulfide bonds in proteins can easily reduce disulfide bonds in proteins, the device can efficiently cleave the disulfide bonds in the proteins. The device for cleaving disulfide bonds in proteins can also prevent redox proteins from being mixed into the protein-containing sample.

In the device for cleaving a disulfide bond in a protein according to one aspect of the present disclosure, for example, a redox enzyme that donates an electron to the redox protein may be further immobilized on the electrode.

This eliminates the need to further add redox enzymes to the protein-containing sample. Therefore, since the device for cleaving disulfide bonds in proteins can more easily reduce disulfide bonds in proteins, the device can efficiently cleave the disulfide bonds in the proteins. The device for cleaving disulfide bonds in proteins can further prevent redox enzymes from being mixed into the protein-containing sample.

In the device for cleaving a disulfide bond in a protein according to one aspect of the present disclosure, for example, a redox molecule that donates an electron to the redox enzyme may be further immobilized on the electrode.

This eliminates the need to further add redox molecules to the protein-containing sample. Therefore, since the device for cleaving disulfide bonds in proteins can more easily reduce disulfide bonds in proteins, the device can efficiently cleave the disulfide bonds in the proteins. The device for cleaving disulfide bonds in proteins can further prevent redox molecules from being mixed into the protein-containing sample.

In the device for cleaving a disulfide bond in a protein according to one aspect of the present disclosure, for example, an electron mediator that donates an electron to the redox molecule may be further immobilized on the electrode.

This eliminates the need to further add electron mediators to the protein-containing sample. Therefore, since the device for cleaving disulfide bonds in proteins can more easily reduce disulfide bonds in proteins, the device can efficiently cleave the disulfide bonds in the proteins. The device for cleaving disulfide bonds in proteins can further prevent electron mediators from being mixed into the protein-containing sample.

In the device for cleaving a disulfide bond in a protein according to one aspect of the present disclosure, for example, an electron mediator that mediates electron transfer between the electrode and the redox protein may be immobilized on the electrode.

With this, because of the increased efficiency of the electron transfer reaction between the electrode and the redox proteins, the device for cleaving disulfide bonds in proteins can efficiently reduce disulfide bonds in proteins with a small amount of redox protein. Accordingly, the device for cleaving disulfide bonds in proteins can efficiently cleave disulfide bonds in proteins.

General or specific aspects of the present disclosure may be realized as a system, a method, a device, an integrated circuit, a computer program, a computer readable medium such as a CD-ROM, or any given combination thereof.

Hereinafter, embodiments are specifically described with reference to the drawings.

Each embodiment described below shows a general or specific example. The numerical values, shapes, materials, components, the arrangement and connection of the components, steps, the processing order of the steps etc., shown in the following embodiments are mere examples, and therefore do not limit the scope of the Claims. Therefore, among the components in the following embodiments, those not recited in any one of the independent claims defining the broadest concept of the present disclosure are described as optional components. The figures are schematic illustrations and are not necessarily precise depictions. Elements that are essentially the same have the same reference signs in the figures, and duplicate description may be omitted or simplified.

The mutually orthogonal X-axis, Y-axis, and Z-axis directions illustrated in the figures will be used as appropriate in the description. In particular, the positive side in the Z-axis direction may be described as the upper side, and the negative side in the Z-axis direction may be described as the lower side.

In the present disclosure, terms indicating relationships between elements such as "parallel" and "perpendicular", terms indicating shapes of elements such as "rectangular", and numerical ranges refer not only to their strict meanings, but encompass a range of essentially equivalents, such as a range of deviations of a few percent.

In the figures of the present disclosure, dashed lines indicate the boundaries of what is not visible from the surface, as well as regions.

### [Embodiment 1]

Hereinafter, Embodiment 1 will be described in detail with reference to FIG. 1 through FIG. 4.

### Device for Cleaving Disulfide Bonds in Proteins

### 1. Overview

First, an overview of the device for cleaving disulfide bonds in proteins according to Embodiment 1 will described with reference to FIG. 1. FIG. 1 illustrates one example of the configuration of device 100a for cleaving disulfide bonds in proteins according to Embodiment 1.

Device 100a for cleaving disulfide bonds in proteins donates electrons to redox proteins that reduce and thus cleave disulfide bonds in proteins to repeatedly activate redox proteins that have lost their reducing power, thereby continuously reducing the disulfide bonds in the proteins. Device 100a for cleaving disulfide bonds in proteins can adjust the transfer rate of electrons and the amount of energy donated from the electrode (for example, working electrode 1a) to the redox proteins by controlling the voltage applied to the electrode (i.e., working electrode 1a) by power supply 20. Proteins that can be applied to such a device may be any protein that has a disulfide bond, for example, a digestion-resistant protein. The digestion-resistant protein may be, for example, at least one of prolamin, egg albumin, or β-lactoglobulin.

As mentioned above, digestion-resistant proteins are proteins that are difficult to digest in the human digestive tract, and are found in plant-based as well as animal-based foods. Digestion-resistant proteins include, for example, fosvitin from egg yolk, prolamin, the major protein of rice, legumin from kidney beans, phazeolin from adzuki beans, gliadin and glutenin from wheat, buckwheat protein from buckwheat, glycinin and conglisinin from soybeans, or cocoa protein from cacao.

For example, digestion-resistant proteins subject to digestibility improvement in Embodiment 1 have disulfide bonds. Disulfide bonds are very strong bonds that are not easily broken by heat, acids, or enzymes (for example, gastric digestive enzymes). Therefore, digestion-resistant proteins with disulfide bonds are difficult to digest in the human digestive tract. When a digestion-resistant protein has a disulfide bond, reducing the digestion-resistant protein means reducing the disulfide bond in the digestion-resistant protein. The reduction of disulfide bonds in proteins will be described in detail later.

### 2. Configuration

Next, the configuration of device 100a for cleaving disulfide bonds in proteins according to Embodiment 1 will be described with reference to FIG. 1 and FIG. 2. FIG. 2 is a block diagram illustrating one example of the functional configuration of device 100a for cleaving disulfide bonds in proteins according to Embodiment 1.

Device 100a for cleaving disulfide bonds in proteins according to Embodiment 1 includes an electrode (for example, working electrode 1a) for donating electrons to redox proteins that reduce and thus cleave disulfide bonds in proteins through the application of voltage, power supply 20 for applying voltage to the electrode (i.e., working electrode 1a), and controller 30 that controls the application of the voltage by power supply 20. The electrode that donates electrons to the redox proteins (hereinafter also simply referred to as working electrode 1a) is a component of voltage applier 10a.

### Voltage Applier

Voltage applier 10a donates electrons from the electrode (working electrode 1a) to the redox proteins. Voltage applier 10a is, for example, a three-electrode cell that includes working electrode 1a, reference electrode 2, counter electrode 3, cell 4, lid 5, terminals 6a, 6b, and 6c, and leads 7a, 7b, and 7c. Voltage applier 10a may be a two-electrode cell that includes, for example, working electrode 1a and counter electrode 3.

Working electrode 1a is an electrode that is sensitive to electrochemical responses to trace components in sample solution 9a at the electrode surface thereof. Counter electrode 3 is an electrode that establishes a potential difference with working electrode 1a. Working electrode 1a and counter electrode 3 are made of a conductive material. The conductive material may be, for example, a carbon material, a conductive polymer material, a semiconductor, or a metal. Carbon material examples include carbon nanotube, Ketjen black (registered trademark), glassy carbon, graphene, fullerene, carbon fiber, carbon fabric, and carbon aerogel. Conductive polymer material examples include polyaniline, polyacetylene, polypyrrole, poly(3,4-ethylenedioxythiophene), poly(p-phenylenevinylene), polythiophene, and poly(p-phenylene sulfide). Semiconductor examples include silicone, germanium, indium tin oxide (ITO), titanium oxide, copper oxide, and silver oxide. Metal examples include gold, platinum, silver, titanium, aluminum, tungsten, copper, iron, and palladium. Here, working electrode 1a is, for example, a glassy carbon electrode, and counter electrode 3 is a platinum electrode. The conductive material is not particularly limited as long as the conductive material is not decomposed by its own oxidation reaction.

Reference electrode 2 is an electrode that does not react with the components in sample solution 9a and maintains a constant potential, and is used to control the potential difference between working electrode 1a and reference electrode 2 to a constant level by power supply 20. Here, reference electrode 2 is a silver/silver chloride electrode.

Cell 4 is a holder for holding sample solution 9a in which proteins are present. Sample solution 9a contains at least proteins and redox proteins that reduce the disulfide bonds in the proteins. In addition to proteins and redox proteins, sample solution 9a may also contain redox enzymes that reduce the redox proteins. In addition to proteins, redox proteins, and redox enzymes, sample solution 9a may also contain redox molecules that reduce the redox enzymes. In addition to proteins, redox proteins, redox enzymes, and redox molecules, sample solution 9a may also contain electron mediators that are involved in the electron transfer between the electrode and the redox proteins. Hereinafter, an example in which sample solution 9a contains proteins, redox proteins, redox enzymes, redox molecules, and electron mediators will be described. As used herein, reducing proteins means reducing the disulfide bonds in proteins.

Next, the components in sample solution 9a will be described with reference to FIG. 3. FIG. 3 is a schematic diagram illustrating the components in sample solution 9a and the electron transfer reactions between them (electrons are denoted as "e⁻" in the figure). An electron transfer reaction is a reaction involving the transfer of electrons, and is also referred to as a redox reaction. Each component present in the reaction system is reduced when it receives an electron and is oxidized when it donates an electron. Therefore, each component has two forms, oxidized (ox) and reduced (red).

For example, as illustrated in FIG. 3, the protein (target moleculeₒₓ in the figure) has a disulfide bond (-S-S-). Among proteins that have disulfide bonds, a digestion-resistant protein includes, for example, prolamin, which is listed above in "1. Overview".

The disulfide bond of the protein (target moleculeₒₓ) is reduced to a thiol group by donation of an electron from a reduced redox protein (redox protein_{red}). Above the target moleculeₒₓ, FIG. 3 schematically illustrates the portion (a) containing the disulfide bond of the protein before reduction. Below the target molecule_{red}, FIG. 3 schematically illustrates the portion (b) corresponding to (a) above in the protein after reduction. In (a) and (b), the white circles are amino acids. As illustrated in (a) and (b), when the disulfide bond of the protein is reduced, the loop portion of the amino acid sequence is unraveled, making it easier for the digestive enzyme to act on the site to be cleaved by the digestive enzyme.

Note that in (a) and (b) above, the loop portion is given as one non-limiting example. Disulfide bonds are bonds that connect the secondary structures of proteins to each other and strengthen the three-dimensional structure of the protein. Therefore, when the disulfide bond is cleaved, the connection between the secondary structures is broken, increasing the degree of freedom (fluctuation) of the three-dimensional structure of the protein. As a result, it easier for digestive enzymes to act on the cleavage sites where the peptide chain of the protein is cleaved by the digestive enzymes, thus improving the digestibility of a protein such as a digestion-resistant protein.

Redox proteins are proteins, polypeptides, or oligopeptides of any size or structure. Redox proteins improve the digestibility of digestion-resistant proteins by reducing the digestion-resistant proteins. For example, redox proteins cleave the disulfide bonds of digestion-resistant proteins by reducing them to thiol groups. As a result, the digestibility of digestion-resistant proteins is improved because the increased degree of freedom (fluctuation) of the conformational structure of digestion-resistant proteins due to disulfide bonds makes them more receptive to digestive enzymes acting thereon, as described above. The redox protein that reduces a disulfide bond is, for example, thioredoxin, glutathione, a protein with at least one thioredoxin-like domain, or a protein with at least one glutathione-like motif. These redox proteins have thiol groups. For example, thioredoxin is a low molecule weight redox protein with an active site motif having an amino acid sequence consisting of Trp (tryptophan)-Cys (cysteine)-Gly (glycine)-Pro (proline)-Cys (cysteine). Thioredoxin comes in two forms, a reduced form and an oxidized form, depending on the redox state of the thiol groups of the two Cys in the active site. Glutathione is a tripeptide consisting of Glu (glutamic acid)-Cys (cysteine)-Gly (glycine). Glutathione reduces a disulfide bond of a digestion-resistant protein to a thiol group through the reducing power of the thiol group of Cys. Reduced glutathione is a tripeptide consisting of the above three amino acids. Oxidized glutathione is a molecule consisting of two molecules of reduced glutathione joined by a disulfide bond.

A protein with at least one thioredoxin-like domain is, for example, a protein with at least one thioredoxin-like domain containing a Cys-AAc₁-AAc₂-Cys active site. AAc1 and AAc2 may be any amino acid residue other than cysteine residue. The number of amino acid residues between the two Cys in the active site is not limited to the two amino acid residues of AAc₁ and AAc₂, and may be three or four, for example. The thioredoxin-like domain may contain at least one Cys-AAc₁-AAc₂-Cys active site. For example, the thioredoxin-like domain may contain a Cys-AAc₁-AAc₂-Cys active site and a Cys-AAc₁'-AAc₂'-Cys active site, and may contain a Cys-AAc₁-AAc₂-Cys-AAc₁'-AAc₂'-Cys active site. AAc₁ and AAc₁' may be the same or different amino acid residues. Additionally, AAc₂ and AAc₂' may be the same or different amino acid residues.

A protein with at least one glutathione-like motif is, for example, a protein with at least one AAc₃-Cys-AAc₄ active site, for example. Stated differently, an AAc₃-Cys-AAc₄ active site is a motif similar to the chemical properties of glutathione (also referred to as a glutathione-like motif). AAc₃ may be the same acidic amino acid as Glu in the glutathione, and AAc₄ may be the same neutral amino acid as Gly in the glutathione. AAc₃ may be, for example, Glu, γ-Glu, Asp (aspartic acid), β-Asp, GluGly, γ-GluGly, AspGly, or β-AspGly. AAc₄ may be, for example, Gly, Phg (phenylglycine), Ala (alanine), β-Ala, or Phe (phenylalanine).

Redox enzymes are enzymes that catalyze the redox reaction of redox proteins. Redox enzymes donate electrons to redox proteins. More specifically, redox enzymes donate electrons donated from working electrode 1a to oxidized redox proteins. Redox enzymes may donate electrons from working electrode 1a via electron mediators, redox proteins, or both electron mediators and redox proteins. The redox enzyme is, for example, (i) an enzyme that catalyzes the reduction of oxidized thioredoxin, including a nicotinamide adenine dinucleotide phosphate (NADPH)-thioredoxin reductase or a ferredoxin-thioredoxin reductase, or (ii) an enzyme that catalyzes the reduction of oxidized glutathione, including a glutathione reductase.

For example, if the redox protein is thioredoxin, the redox enzyme is an enzyme that catalyzes the reduction of oxidized thioredoxin, including NADPH-thioredoxin reductase or ferredoxin-thioredoxin reductase. Enzymes that catalyze the reduction of oxidized thioredoxin are, for example, polypeptides or proteins with thioredoxin reducing activity. Such an enzyme may be, for example, an NADPH-thioredoxin reductase or a ferredoxin-thioredoxin reductase, or a mutant enzyme in which a portion of the amino acid sequence of the NADPH-thioredoxin reductase or the ferredoxin-thioredoxin reductase is mutated. The enzyme may be a metalloenzyme containing metal atoms such as iron, chromium, manganese, magnesium, calcium, cobalt, molybdenum, zinc, copper, or nickel in the active site of the NADPH-thioredoxin reductase or the ferredoxin-thioredoxin reductase. The enzyme may be a hybrid enzyme in which thioredoxin is linked to the C-terminal side of the NADPH-thioredoxin reductase or the ferredoxin-thioredoxin reductase.

For example, if the redox protein is glutathione, the redox enzyme is an enzyme that catalyzes the reduction of oxidized glutathione, including glutathione reductase. Enzymes that catalyze the reduction of oxidized glutathione are, for example, polypeptides or proteins with glutathione reducing activity. Such an enzyme may be, for example, a riboflavin-dependent glutathione reductase, such as flavin adenine dinucleotide (FAD) or flavin mononucleotide (FMN), or a NADPH-dependent glutathione reductase.

Redox molecules are molecules that reduce redox enzymes. Redox molecules reduce redox enzymes by donating electrons to the redox enzymes. More specifically, redox molecules reduce redox enzymes by donating electrons donated by working electrode 1a to the redox enzymes. Redox molecules may donate electrons from working electrode 1a via electron mediators. The redox molecule is, for example, nicotinamide adenine dinucleotide phosphate (NADPH) or ferredoxin. The redox molecule may be nicotinamide adenine dinucleotide (NADH).

Electron mediators are redox substances that mediate electron transfer between the electrode and the redox proteins. Electron mediators donate electrons to redox molecules. More specifically, electron mediators donate electrons donated from working electrode 1a to redox molecules. The electron mediators may donate electrons directly to oxidized redox proteins. The electron mediator is not particularly limited as long as it enables electron transfer between the electrode and the redox proteins. The electron mediator may be selected according to the redox potential of the target molecule to which the electron mediator donates electrons. The electron mediator may be, for example, a compound with a bipyridine skeleton, a compound with a quinone skeleton, or a compound with a phenylenediamine skeleton. These compounds may be used alone, and, alternatively, a combination of two or more of these compounds may be used.

A compound with a bipyridine skeleton may be, for example, a compound with a 2,2'-bipyridine skeleton, a compound with a 2,4'-bipyridine skeleton, a compound with a 4,4'-bipyridine skeleton, or a derivative of these (for example, a 4,4'-bipyridinium derivative). A compound with a bipyridine skeleton may be a bipyridine compound with substituents on the bipyridine skeleton (i.e., a bipyridine derivative) or a bipyridine compound without substituents. Substituents include hydrogen, halogen, hydroxyl, nitro, carboxyl, carbonyl, amino, amide, or sulfonic acid groups, or alkyl, aryl, heteroaromatic alkyl, or phenyl groups substituted therewith. An aromatic ring may be formed between two adjacent substituents. The substituents are the same for a compound with a quinone skeleton and a compound with a phenylenediamine skeleton, as described below. When the electron mediator is a compound with a bipyridine skeleton, the electron mediator is, for example, 1,1'-dimethyl-4,4'-bipyridinium (methyl viologen), 1-methyl-1'-carboxylmethyl-4,4'-bipyridinium, 1,1'-dicarboxymethyl-4,4'-bipyridinium, 1-methyl-1'-aminoethyl-4,4'-bipyridinium, 1,1'-diaminoethyl-4,4'-bipyridinium, 1-methyl-1'-ethyl-4,4'-bipyridinium, 1-methyl-1'-propyl-4,4'-bipyridinium, 1-methyl-1'-butyl-4,4'- bi pyridinium, 1-methyl-1'-pentylhexyl-4,4'-bipyridinium, 1-methyl-1'-hexyl-4,4'-bipyridinium, 1-methyl-1'-heptyl-4,4'-bipyridinium, 1-methyl-1'-octyl-4,4'-bipyridinium, 1-methyl-1'-nonyl-4,4'-bipyridinium, or 1-methyl-1'-decyl-4,4'-bipyridinium, or a compound in which the methyl group at position 1 of these compounds is replaced with an ethyl group.

A compound with a quinone skeleton may be, for example, a compound with a benzoquinone skeleton, a compound with a naphthoquinone skeleton, a compound with an anthraquinone skeleton, or a derivative of these. A compound with a quinone skeleton may or may not have substituents. As substituents have been discussed above, description here will be omitted. When the electron mediator is a compound with a quinone skeleton, the electron mediator may be, for example, methylbenzoquinone, dimethylbenzoquinone (such as 2,5-dimethyl-1,4-benzoquinone, 2,3-dimethyl-1,4-benzoquinone, and 2,6-dimethyl-1,4-benzoquinone), 2,3-dichloro-5,6-dicyano-1,4-benzoquinone, 2,3,5,6-tetramethyl-1,4- benzoquinone, 2,3,5,6-tetrachloro-1,4-benzoquinone (chloranil), ubiquinone (CoQ), pyrroloquinoline quinone (PQQ), 1,2-naphthoquinone-4-sulfonic acid, 2-methyl-1,4-naphthoquinone (vitamin K₃), 2-hydroxy-1,4-naphthoquinone, 1,2-dihydroxyanthraquinone (alizarin), 1,2,4-trihydroxyanthraquinone (purpurin), or 9,10-phenanthrenequinone. The electron mediator may be, for example, a benzenediol in which the ketone group of the benzoquinone skeleton is replaced with a hydroxyl group, or more specifically, hydroquinone in which the ketone group of 1,4-benzoquinone is replaced with a hydroxyl group (1,4-benzenediol), or resorcinol in which the ketone group of 1,3-benzoquinone is replaced with a hydroxyl group (1,3-benzenediol).

A compound with a phenylenediamine skeleton may or may not have substituents, for example. When the electron mediator is a compound with a phenylenediamine skeleton, the electron mediator may be, for example, p-phenylenediamine, 2,3,5,6-tetramethyl-1,4-phenylenediamine, N,N-dimethyl-p-phenylenediamine, N,N'-diphenyl-p-phenylenediamine (DPPD), N - isopropyl-N'-phenyl-p-phenylenediamine (IPPD), or N -(1,3-dimethylbutyl)-N'-phenyl-p-phenylenediamine (6PPD).

Referring again to FIG. 1, terminal 6a, terminal 6b, and terminal 6c that electrically connect working electrode 1a, reference electrode 2, and counter electrode 3 to power supply 20, respectively, are arranged on lid 5. Leads extend from each terminal, connecting the terminals to a battery. Working electrode 1a is connected to terminal 6a via lead 7a, reference electrode 2 is connected to terminal 6b via lead 7b, and counter electrode 3 is connected to terminal 6c via lead 7c.

### Power Supply

Power supply 20 applies voltage to an electrode (working electrode 1a). More specifically, power supply 20 applies voltage between working electrode 1a and counter electrode 3 of voltage applier 10a and controls the potential difference between working electrode 1a and reference electrode 2 to a predetermined value in accordance with a control signal output from controller 30. For example, power supply 20 may apply voltage so that the voltage applied to working electrode 1a is between -1.0 V and 0 V, inclusive, with reference electrode 2 as the reference (0 V). Here, reference electrode 2 is, for example, a silver/silver chloride electrode.

As illustrated in FIG. 2, power supply 20 includes, for example, obtainer 21, information processor 22, and voltage controller 23.

Obtainer 21 obtains a control signal output from controller 30 and outputs the obtained control signal to information processor 22. Obtainer 21 may also obtain measurement data such as the potential of each electrode in voltage applier 10a and the current value flowing in sample solution 9a.

Information processor 22 processes the information obtained by obtainer 21. For example, when information processor 22 obtains a control signal from obtainer 21, information processor 22 outputs the obtained control signal to voltage controller 23. When voltage controller 23 starts applying voltage to the electrodes in voltage applier 10a, information processor 22 obtains measurement data such as the potential of each electrode in voltage applier 10a and the current value flowing in sample solution 9a, which are obtained from obtainer 21, and derives the voltage to be applied to working electrode 1a based on the obtained measurement data such that the potential difference between working electrode 1a and reference electrode 2 is maintained at a predetermined value. Information processor 22 outputs, to voltage controller 23, a control signal that controls the voltage of working electrode 1a with the derived voltage.

Voltage controller 23 applies voltage to each electrode of voltage applier 10a based on the control signal output from information processor 22.

Although FIG. 1 illustrates an example where power supply 20 and controller 30 are separate units, power supply 20 may include controller 30.

### Controller

Controller 30 processes information for controlling the application of voltage by power supply 20. Controller 30 is realized, for example, by a processor, a microcomputer, or dedicated circuitry. FIG. 1 illustrates an example where controller 30 is a computer.

Controller 30 includes, for example, obtainer 31, information processor 32, storage 33, and outputter 34.

Obtainer 31 obtains, for example, information related to instructions input by the user (hereinafter referred to as "instruction information"), and outputs the obtained instruction information to information processor 32.

Information processor 32 derives, for example, the conditions under which voltage is to be applied to each electrode of voltage applier 10a (also called voltage application conditions) based on the instruction information obtained by obtainer 31. The instruction information may be, for example, the type of protein, the amount of sample solution 9a, the amount of time until completion of the process, the time of completion, or the degree of reduction (in percentage or a five-step display, for example).

Information processor 32 may output a control signal to power supply 20 to control voltage application under the conditions derived based on the instruction information, and, alternatively, may output a control signal to power supply 20 to control voltage application under voltage application conditions set in advance by the user.

Outputter 34 outputs the control signal obtained from information processor 32 to power supply 20.

Storage 33 stores data, such as the instruction information, obtained by obtainer 31 and computer programs (for example, an application program for controlling power supply 20) executed by controller 30.

### 3. Operation

Next, the operation of device 100a for cleaving disulfide bonds in proteins according to Embodiment 1 will be described with reference to FIG. 1 through FIG. 4. FIG. 4 is a flowchart illustrating one example of the operation of device 100a for cleaving disulfide bonds in proteins according to Embodiment 1.

First, the preparation process (not illustrated in the drawings) performed before operating device 100a for cleaving disulfide bonds in proteins will be described. For example, the preparation process may be performed by the user. In the preparation process, first, sample solution 9a is prepared. The user introduces a protein-containing sample into cell 4 of voltage applier 10a. Next, sample solution 9a is prepared by adding redox proteins, redox enzymes, redox molecules, and electron mediators to the sample in cell 4. The disulfide bonds in proteins present in sample solution 9a are cleaved by being reduced by the reduced redox proteins. The added redox proteins, redox enzymes, redox molecules, and electron mediators may all be in reduced form or a mixture of oxidized and reduced forms.

Next, the user inserts the electrodes into sample solution 9a and sets the electrodes in place. The electrodes are specifically working electrode 1a, reference electrode 2, and counter electrode 3. Working electrode 1a is connected to lead 7a extending from terminal 6a arranged on lid 5, reference electrode 2 is connected to lead 7b extending from terminal 6b arranged on lid 5, and counter electrode 3 is connected to lead 7c extending from terminal 6c arranged on lid 5.

Next, the user inputs, to device 100a for cleaving disulfide bonds in proteins, information related to instructions (i.e., the instruction information), such as the type of protein, the amount of sample solution 9a, the amount of time until completion of the process, the completion time, or the degree of reduction.

In the above preparation process, the user prepared sample solution 9a by adding, to the protein-containing sample, redox proteins, redox enzymes, redox molecules, and electron mediators in reduced form only or in a mixture of reduced and oxidized forms, but oxidized redox proteins, oxidized redox enzymes, oxidized redox molecules, and oxidized electron mediators may also added. This reduces variation in reduction efficiency because the proteins (more specifically, the disulfide bonds in the proteins) present in sample solution 9a are reduced after the application of voltage is started in step S102.

In the above preparation process, sample solution 9a is prepared by introducing a protein-containing sample and the like into cell 4, but a pre-prepared sample solution 9a may be introduced into cell 4.

Next, the operation of device 100a for cleaving disulfide bonds in proteins will be described. After the instruction information is input by the user, controller 30 sets the conditions for applying voltage to each electrode of voltage applier 10a (step S101). In setting the conditions, controller 30 derives the voltage application conditions based on the input instruction information and outputs, to power supply 20, a control signal that controls the voltage application by power supply 20 under the derived voltage application conditions. In step S101, the user may select a program number associated with the voltage application conditions, and controller 30 may obtain the program number and set the voltage application conditions.

Next, power supply 20 obtains the control signal output from controller 30 and starts applying voltage to the electrodes in accordance with the obtained control signal (step S102). For example, power supply 20 applies a voltage between working electrode 1a and counter electrode 3 of voltage applier 10a to control the potential difference between working electrode 1a and reference electrode 2 to a predetermined value (for example, a value in the range between -1.0 V and 0 V, inclusive). In other words, power supply 20 applies voltage so that the voltage applied to working electrode 1a is between -1.0 V and 0 V, inclusive, with reference electrode 2 as the reference (0 V). Here, reference electrode 2 is, for example, a silver/silver chloride electrode. This causes electrons to be donated from working electrode 1a to oxidized redox proteins in sample solution 9a. As a result, the oxidized redox proteins are reduced to reduced redox proteins (step S103). Next, the reduced redox proteins cleave the S-S bonds (disulfide bonds) in the proteins in sample solution 9a bonds by reducing them (step S104). Step S103 and step S104 are repeated while voltage is applied to the electrodes from power supply 20. In step S103, the redox enzymes, redox molecules, and electron mediators in sample solution 9a are also reduced from their oxidized forms to their reduced forms.

Next, controller 30 determines whether processing under the set conditions is complete (step S105). The conditions set are, for example, the duration (time) of the voltage application or the number of times the voltage is applied (for example, pulsed voltage). If controller 30 determines that the processing under the set conditions is not complete (No in step S105), controller 30 causes power supply 20 to continue applying voltage (step S106). Steps S103 and S104 are then repeated until the next decision (step S105) is made. However, if controller 30 determines that processing under the set conditions is complete (Yes in step S105), controller 30 causes power supply 20 to end the application of voltage (step S107). This completes the process of cleaving the disulfide bonds in the proteins in sample solution 9a.

Although the above preparation process is exemplified as performed by the user, the preparation process may be performed by device 100a for cleaving disulfide bonds in proteins. In such cases, device 100a for cleaving disulfide bonds in proteins may further include an introducer (not illustrated in the drawings), a collector (not illustrated in the drawings), an introduction port (not illustrated in the drawings), and an outlet port (not illustrated in the drawings). For example, the introducer may introduce the protein-containing sample, redox proteins, redox enzymes, redox molecules, and electron mediators into cell 4 through an introduction port in cell 4. For example, the collector may collect the sample solution 9a marked by improved protein digestibility out of cell 4 through an outlet port in cell 4.

### [Embodiment 2]

Next, Embodiment 2 will be described in detail with reference to FIG. 5 and FIG. 6. In Embodiment 2, description will focus on the points of difference from Embodiment 1. Description of content that overlaps with Embodiment 1 will be simplified or omitted.

### Device for Cleaving Disulfide Bond in Protein

### 1. Overview

First, an overview of the device for cleaving disulfide bonds in proteins according to Embodiment 2 will described with reference to FIG. 5. FIG. 5 illustrates one example of the configuration of device 100b for cleaving disulfide bonds in proteins according to Embodiment 2.

Device 100b for cleaving disulfide bonds in proteins differs from Embodiment 1 in that the redox proteins that reduce the digestion-resistant proteins are immobilized on the electrode (in this case, working electrode 1b), so there is no need to prepare a sample solution. Device 100b for cleaving disulfide bonds in proteins is a device that donates electrons to redox proteins that cleave disulfide bonds in proteins present in sample 9b by reducing the disulfide bonds, to repeatedly activate redox proteins that have lost their reducing power on an electrode and thus continuously cleaved the disulfide bonds in the proteins. Stated differently, device 100b for cleaving disulfide bonds in proteins can more conveniently cleave disulfide bonds in proteins because the electrode can be inserted directly into the sample to reduce the disulfide bonds in the proteins. Moreover, since device 100b for cleaving disulfide bonds in proteins does not require adding redox proteins or other substances to the protein-containing sample, it can prevent redox proteins or other substances (i.e., substances not originally contained in the sample) from being mixed into the sample.

### 2. Configuration

Next, the configuration of device 100b for cleaving disulfide bonds in proteins according to Embodiment 2 will described with reference to FIG. 5.

As illustrated in FIG. 5, device 100b for cleaving disulfide bonds in proteins according to Embodiment 2 includes voltage applier 10b, power supply 20, and controller 30. Device 100b for cleaving disulfide bonds in proteins differs from Embodiment 1 in that it includes working electrode 1b with redox proteins immobilized on the electrode surface thereof.

For example, redox proteins, redox enzymes, redox molecules, and electron mediators may all be immobilized on the electrode, as long as at least the redox proteins are immobilized on the electrode. This prevents redox proteins, redox enzymes, redox molecules, and electron mediators from remaining in the protein-containing sample.

### Working Electrode

Working electrode 1b is an electrode for donating electrons to redox proteins by voltage application. The redox proteins are immobilized on working electrode 1b. Furthermore, in addition to the redox proteins, redox enzymes that donate electrons to the redox proteins may be immobilized on working electrode 1b, redox enzymes and redox molecules that donate electrons to the redox enzymes may be immobilized on working electrode 1b, and redox enzymes, redox molecules, and electron mediators that donate electrons to the redox molecules may be immobilized on working electrode 1b. Furthermore, in addition to the redox proteins, electron mediators may be immobilized on working electrode 1b. Hereinafter, an example in which redox proteins, redox enzymes, redox molecules, and electron mediators are immobilized on the surface of working electrode 1b will be described.

FIG. 6 is a schematic cross-sectional view taken at line VI-VI of working electrode 1b illustrated in FIG. 5. As illustrated in FIG. 6, working electrode 1b includes base electrode 18, and redox proteins 14, redox enzymes 15, redox molecules 16, and electron mediators 17 immobilized on base electrode 18. Base electrode 18 includes substrate 11 made of electrode material, conductive polymer 12, and conductive particles 13. Since redox proteins 14, redox enzymes 15, redox molecules 16, and electron mediators 17 (hereinafter also collectively referred to as "redox protein 14, etc.") have been mentioned above, repeated description will be omitted here.

Substrate 11 is made of, for example, a porous electrode material. From the viewpoint of strength, stiffness, and light weight, the conductive material may be, for example, carbon fiber, such as polyacrylonitrile (PAN)-based carbon fiber, pitch-based carbon fiber, or rayon-based carbon fiber. Among these, from the viewpoint of mechanical strength of the porous electrode material, the conductive material may be PAN-based carbon fiber. PAN-based carbon fiber may be, for example, a short-fiber randomly oriented mat formed of TORAYCA (registered trademark) yarn. One type of carbon fiber may be used, or several different types of carbon fiber may be used. Other known fibers, such as glass fiber, aramid fiber, polyethylene terephthalate fiber, vinylon fiber, polyester fiber, amide fiber, or ceramic fiber may also be used with the carbon fiber.

Conductive polymer 12 is not limited so long as it is a polymer having conductive properties, and may be, for example, polyacetylene, polythiophene, polyfluorene, polyethylenevinylene, polyphenylenevinylene, polypyrrole, or polyaniline. Conductive polymer 12 may contain a dopant.

Conductive particles 13 are not limited so long as they are particles that behave as good electrical conductors. For example, conductive particles 13 may be conductive polymer particles such as polyacetylene particles, polyaniline particles, polypyrrole particles, polythiophene particles, polyisothianaphthene particles, and polyethylene dioxithiophene particles, and may be carbon particles, carbon fiber particles, or metal particles. Among these, conductive particles 13 may be carbon or metal particles, as they exhibit high conductivity and stability.

Carbon particles may be carbon nanofibers, including carbon black, expanded graphite, scale graphite, graphite powder, graphite particles, graphene sheets, carbon milled fiber, carbon nanotubes, and vapor-phase grown carbon fiber (VGCF; registered trademark). Among these, carbon black and carbon milled fiber may be used as they exhibit high conductivity and are inexpensive. Carbon black may be furnace black, acetylene black, thermal black, channel black, or Ketjen black (registered trademark).

The metal particles are not particularly limited, but when carbon fiber is used as a reinforcing fiber, particles of platinum, gold, silver, copper, tin, nickel, titanium, cobalt, zinc, iron, chromium, aluminum, particles of alloys mainly composed of these metals, tin oxide, indium oxide, and indium tin oxide (ITO) are acceptable because they prevent corrosion due to potential difference with the carbon fiber.

The shape of conductive particles 13 is not particularly limited, and may be spherical, non-spherical, or porous particles. From the viewpoint of forming conductive bridges between carbon fiber layers, it is desirable to have a large aspect ratio.

The method of immobilizing redox proteins 14, etc., onto the surface of the electrode is not particularly limited; possible methods include, for example, chemically immobilizing redox proteins 14, etc., onto the electrode, indirectly immobilizing redox proteins 14, etc., onto the electrode using a conductive polymer or cross-linking agent, and immobilizing redox proteins 14, etc., onto the electrode via monolayer-forming molecules. In the example in FIG. 6, conductive polymers are used to immobilize redox proteins 14, etc., onto the electrode.

### 3. Operation

The operation of device 100b for cleaving disulfide bonds in proteins according to Embodiment 2 is substantially the same as the operation of device 100a for cleaving disulfide bonds in proteins according to Embodiment 1, so the flow of the implementation example will be omitted. Embodiment 2 differs from the operation of device 100a for cleaving disulfide bonds in proteins according to Embodiment 1 in that it reduces oxidized redox proteins immobilized on working electrode 1b (specifically, step S103). Additionally, the preparation process does not require a process for preparing a sample solution.

More specifically, the inventors discovered a method for continuously reducing and cleaving disulfide bonds in proteins by causing device 100b for cleaving disulfide bonds in proteins to repeatedly reduce oxidized redox proteins (for example, oxidized thioredoxin) to reduced redox proteins (for example, reduced thioredoxin). With this, device 100b for cleaving disulfide bonds in proteins can efficiently reduce proteins using a smaller amount of redox protein than is required to reduce disulfide bonds in all proteins. As a result, device 100b for cleaving disulfide bonds in proteins can efficiently cleave disulfide bonds in proteins.

### [Variation of Embodiment 2]

Embodiment 2 describes an example in which at least redox proteins are immobilized on working electrode 1b, but in this variation of Embodiment 2, redox proteins do not need to be immobilized on working electrode 1b. For example, electron mediators that mediate the electron transfer between the working electrode and the redox proteins should be immobilized on the working electrode. Here, the sample solution includes proteins and redox proteins. This improves the reduction efficiency of disulfide bonds in proteins since electron transfer efficiency is improved compared to when the redox proteins receive electrons directly from the working electrode. As a result, the device for cleaving disulfide bonds in proteins according to this variation of Embodiment 2 can efficiently cleave disulfide bonds in proteins.

### Implementation Examples

Hereinafter, implementation examples of the method for cleaving disulfide bonds in proteins according to the present disclosure will be described in detail, but the following implementation examples are nothing more than examples, and the present disclosure is not limited to the following implementation examples in any way.

In the following implementation examples, the reduction of disulfide bonds in proteins with and without the application of voltage to a sample solution containing proteins was verified. The proteins used were the digestion-resistant proteins prolamin, milk globulin, and egg albumin.

In the following implementation examples, a very small amount of redox protein was used relative to the number of disulfide bonds in the proteins.

The redox enzymes and redox proteins used below are ferredoxin-thioredoxin reductase and thioredoxin, and were prepared using the method disclosed in Non-patent Literature (NPL) 1 (Keisuke Yoshida et al., "Distinct electron transfer from ferredoxin-thioredoxin reductase to multiple thioredoxin isoforms in chloroplasts", Biochemical Journal, Portland Press, 2017, Vol. 474 (Pt. 8), pp. 1347-1360).

The following describes (1) the reduction of disulfide bonds in prolamin and the improvement of prolamin digestibility by reduction of disulfide bonds, (2) the reduction of disulfide bonds in milk globulin, and (3) the reduction of disulfide bonds in egg albumin.

In the following implementation examples, redox proteins are not immobilized on the electrode surface, but are included in the sample solution along with the proteins. In this case, the redox proteins do not receive electrons directly from the electrode by electron transfer at the interface, but rather indirectly from the electrode via electron mediators, electron transfer molecules (i.e., redox molecules), or electron transfer enzymes (i.e., redox enzymes). Hereafter, this is referred to as redox protein reduction by indirect electron transfer.

### (1) Reduction of Disulfide Bonds in Prolamin and Improvement of Prolamin Digestibility by Reduction of Disulfide Bonds

First, the reduction of disulfide bonds in digestion-resistant proteins and the improvement of digestibility of digestion-resistant proteins by the reduction were verified using prolamin.

### Comparative Example 1 and Comparative Example 2

In Comparative Example 1, Sample Solution I was allowed to stand overnight at a low temperature without voltage applied, and then Sample Solution I was incubated under predetermined conditions, without adding digestive enzymes.

In Comparative Example 2, Sample Solution I was allowed to stand overnight at a low temperature without voltage applied, and then digestive enzymes were added, and Sample Solution I was incubated under predetermined conditions. Hereinafter, Comparative Example 1 and Comparative Example 2 will be described in detail.

### Preparation of Sample Solution I

Sample Solution I was prepared by adding, to porridge, redox proteins in oxidized form (i.e., oxidized redox proteins) that reduce disulfide bonds in prolamin, redox enzymes that reduce the oxidized redox proteins, coenzymes (i.e., redox molecules) in oxidized form that activate the redox enzymes (hereinafter referred to as "oxidized redox molecules"), and electron mediators that donate electrons to the oxidized redox molecules.

### Reduction of Disulfide Bonds in Prolamin

After Sample Solution I was prepared, Sample Solution I was allowed to stand overnight at a low temperature (for example, the internal temperature of a refrigerator). Voltage was not applied to Sample Solution I.

### Prolamin Degradation via Digestive Enzymes

In Comparative Example I, after Sample Solution I was allowed to stand overnight, and Sample Solution I was incubated at 37°C for 2 hours, without adding digestive enzymes. In Comparative Example 2, after Sample Solution I was allowed to stand overnight, digestive enzymes were added to Sample Solution I, and Sample Solution I was incubated at 37°C for 2 hours. Incubated Sample Solution I of Comparative Example 1 and Comparative Example 2 and molecular weight markers were then electrophoresed by sodium dodecyl sulfate-Polyaclamide gel electrophoresis (SDS-PAGE) using the usual method. Next, the electrophoresed gel was stained, and the density of prolamin bands was quantified by image analysis. The electrophoresis images are illustrated in FIG. 7. FIG. 7 illustrates electrophoresis images after SDS-PAGE in Comparative Example 1, Comparative Example 2, and Implementation Example 1. In FIG. 7, (a) illustrates the electrophoresis images for Comparative Example 1, in which bands of proteins characteristic of cooked rice (proglutelin (57 kD), glutelin α (37 through 39 kD), globulin (26 kD), glutelin β (22 through 23 kD), and prolamin (13 kD and 16 kD)) were observed. In FIG. 7, (b) illustrates the electrophoresis images for Comparative Example 2, in which bands of prolamin (13kD and 16kD) were observed. The electrophoresis images of the molecular weight markers are illustrated in (d) in FIG. 7.

### Calculation of Prolamin Digestion Rate

The values indicating the concentration of the prolamin bands in Sample Solution I of Comparative Example 1 and Sample Solution I of Comparative Example 2, respectively, were calculated. Then, the retention rate of prolamin in Comparative Example 2 was calculated by proportional calculation, using the value indicating the density of the prolamin band in Comparative Example 1 as 100% prolamin retention. As a result, in Comparative Example 2, the prolamin retention rate was 82%. The degradation rate of prolamin in Sample Solution I of Comparative Example 2 was thus 18%. The degradation calculation results are illustrated in FIG. 8. FIG. 8 is a graph illustrating the digestion rate of prolamin after treatment with digestive enzymes in Comparative Example 1, Comparative Example 2, and Implementation Example 1.

### Implementation Example 1

In Implementation Example 1, Sample Solution I was applied with a predetermined voltage overnight, at a low temperature, and then the digestive enzymes were added and Sample Solution I was incubated under predetermined conditions. Sample Solution I was prepared the same as in Comparative Example 1 and Comparative Example 2.

### Reduction of Disulfide Bonds in Prolamin

In Implementation Example 1, a predetermined voltage was applied to Sample Solution I overnight, at a low temperature, using a three-electrode voltage-applying cell (for example, voltage applier 10a in FIG. 1) and a potentiostat (for example, power supply 20 in FIG. 1). Among the three electrodes, a glassy carbon electrode was used for working electrode 1a and an Ag/AgCl electrode was used for reference electrode 2. The predetermined voltage applied to Sample Solution I was controlled by the potentiostat so that the potential of working electrode 1a relative to reference electrode 2 was equal to the reduction potential of the electron mediator.

### Prolamin Degradation via Digestive Enzymes

Next, Sample Solution I was collected after the voltage was applied, digestive enzymes were added to the collected Sample Solution I, and Sample Solution I was incubated at 37 °C for 2 hours. Incubated Sample Solution I and molecular weight markers were electrophoresed by SDS-PAGE, just as in Comparative Example 1 and Comparative Example 2. Staining of the gel after electrophoresis showed no bands of prolamin. The electrophoresis images are illustrated in FIG. 7. The electrophoresis images of Implementation Example 1 are illustrated in (c) in FIG. 7; no prolamin bands were observed at the molecular weights 13 kD and 16kD surrounded by the dashed line.

### Calculation of Prolamin Degradation Rate

Just like in Comparative Example 1 and Comparative Example 2, the value indicating the concentration of the prolamin bands in Sample Solution I of Implementation Example 1 were calculated. Then, the retention rate of prolamin in Implementation Example 1 was calculated by proportional calculation, using the value indicating the density of the band in Comparative Example 1 as 100% prolamin retention. As a result, in Implementation Example 1, the retention rate of prolamin in Sample Solution I was 0%.

Next, the digestion rate of prolamin in Implementation Example 1 was calculated by subtracting the retention rate of Implementation Example 1 (0%) from the retention rate of Comparative Example 1 (100%). The degradation rate calculation results are illustrated in FIG. 8. As illustrated in FIG. 8, in Implementation Example 1, the digestion rate of prolamin in Sample Solution I was 100%.

### Observations

In Comparative Example 1, no voltage was applied to Sample Solution I and no digestive enzymes were added. As a result, as illustrated in (a) in FIG. 7, all of the multiple proteins in Sample Solution I remained. In Comparative Example 2, just as in Comparative Example 1, no voltage was applied to Sample Solution I. However, unlike in Comparative Example 1, digestive enzymes were added to Sample Solution I. As a result, in Comparative Example 2, the digestive enzymes digested all the proteins in Sample Solution I except for prolamin; approximately 20% of the prolamin in Sample Solution I was digested.

These results confirm that when no voltage is applied to Sample Solution I, all the proteins except prolamin are digested by the digestive enzymes, but prolamin is not easily digested by the digestive enzymes.

On the other hand, in Implementation Example 1, the digestive enzymes were added after voltage was applied to Sample Solution I under the predetermined conditions. As a result, in Implementation Example 1, all of the prolamin in Sample Solution I was digested. The difference between Comparative Example 2 and Implementation Example 1 is the application of voltage to Sample Solution I. It is therefore thought that the application of voltage to Sample Solution I reduced and cleaved the disulfide bonds in prolamin, making it easier for the digestive enzymes to act on the prolamin in Sample Solution I, i.e., improving the digestibility of the prolamin.

As described above, Sample Solution I contains redox proteins, redox enzymes, redox molecules, and electron mediators in addition to prolamin. When voltage is applied to Sample Solution I, redox proteins indirectly receive electrons from the electrode via electron mediators, redox molecules, or redox enzymes. Redox proteins are reduced from their oxidized form to their reduced form when they receive an electron. Reduced redox proteins, for example, are themselves oxidized and thus converted to oxidized redox proteins by reducing the disulfide bonds in prolamins. Oxidized redox proteins cannot reduce disulfide bonds, but are believed to reduce to redox proteins in reduced form by indirect electron transfer from the electrode when voltage is applied to Sample Solution I.

The above results suggest that when no voltage is applied to Sample Solution I, oxidized redox proteins in Sample Solution I are not reduced to reduced redox proteins. Stated differently, this suggests that if no voltage is applied to Sample Solution I, once a reduced redox protein in Sample Solution I is oxidized, it cannot be reduced again to a reduced redox protein. Thus, in Comparative Example 2, the digestibility of prolamin was not improved because only enough disulfide bonds were cleaved to reduce the reduced redox proteins in Sample Solution I.

However, the results of Implementation Example 1 suggest that oxidized redox proteins in Sample Solution I are reduced to reduced redox proteins when voltage is applied to the sample solution. Stated differently, this suggests that when voltage is applied to Sample Solution I, once a reduced redox protein in Sample Solution I is oxidized, the oxidized redox protein is reduced again to a reduced redox protein by indirect electron transfer from the electrode. In Implementation Example 1, the digestion rate of prolamin by the digestive enzymes is 100%, suggesting that the application of voltage to Sample Solution I repeatedly reduces the oxidized redox proteins via the mechanism described above, and the reduced redox proteins may continuously cleave the disulfide bonds of prolamin. Therefore, it is believed that with the method for cleaving disulfide bonds in proteins according to the present disclosure, by applying voltage to Sample Solution I, electrons are donated from the electrode to the oxidized redox proteins in Sample Solution I, and the oxidized redox proteins are reduced to reduced redox proteins. Accordingly, with the method for cleaving disulfide bonds in proteins according to the present disclosure, the disulfide bonds in the digestion-resistant proteins (in this case, prolamin) in Sample Solution I are efficiently reduced, and consequently the disulfide bonds in the digestion-resistant protein are efficiently cleaved. Therefore, the method for cleaving disulfide bonds in proteins according to the present disclosure is believed to efficiently improve the digestibility of digestion-resistant proteins present in Sample Solution I.

Regarding the increased digestion rate of prolamin by digestive enzymes, it is believed that the disulfide bonds in prolamin in Sample Solution I were cleaved by reduction by the reductive redox enzymes, and thus made it easier for the digestive enzymes to act on the prolamin. More specifically, the increased digestion rate of prolamin by digestive enzymes is believed to be due to the increased degree of freedom (i.e., fluctuation) of the conformational structure of prolamin due to the cleavage by reduction of the disulfide bonds that connect the secondary structures of prolamin. Therefore, the method for cleaving disulfide bonds in proteins according to the present disclosure is believed to improve digestion of digestion-resistant proteins by digestive enzymes since it can efficiently reduce disulfide bonds in digestion-resistant proteins.

### (2) Reduction of Disulfide Bonds in Milk Globulin

Next, the reduction of disulfide bonds in digestion-resistant proteins was verified using milk globulin.

### Comparative Example 3

In Comparative Example 3, Sample Solution II was allowed to stand overnight at a low temperature without voltage applied, then 10 mM of 4-acetamido-4-maleimidyl-stilbene-2,2-disulfonate (AMS) was added, Sample Solution II was incubated at 37°C for one hour, and SDS-PAGE was performed. AMS is a low molecular weight compound that specifically binds to thiol groups and is used as a thiol group modifying reagent. AMS specifically binds only to thiol groups, resulting in an increase in molecular weight corresponding to the number of thiol groups. Therefore, the number of AMS compounds that bind differs between proteins that have undergone oxidative modification (i.e., proteins whose disulfide bonds have not been reduced) and proteins that have not undergone oxidative modification (i.e., proteins whose disulfide bonds have been reduced). This difference in number can be detected as a difference in mobility with SDS-PAGE.

### Preparation of Sample Solution II

Sample Solution II was prepared by dissolving, in phosphate buffered saline (PBS) at pH 7.4: the target molecules, i.e., oxidized milk globulin; oxidized redox proteins (i.e., inactive redox proteins); redox enzymes that reduce the oxidized redox proteins; and electron mediators that donate electrons to the redox enzymes.

### Reduction of Oxidized Milk Globulin

After Sample Solution II was prepared, Sample Solution II was allowed to stand for 16 hours at a low temperature (for example, the internal temperature of a refrigerator). Voltage was not applied to Sample Solution I.

### Modification of Reduced Proteins with Thiol Group Modifying Reagents

After Sample Solution II was allowed to stand for 16 hours, 10 mM of AMS was added to Sample Solution II and Sample Solution II was incubated at 37°C for 1 hour. Next, the incubated Sample Solution II and molecular weight markers were electrophoresed by a typical SDS-PAGE method. Next, the gel was stained after eletrophoresis. The electrophoresis images are illustrated in FIG. 9. FIG. 9 illustrates electrophoresis images after SDS-PAGE in Comparative Example 3 and Implementation Example 2. In FIG. 9, (a) illustrates electrophoresis images for Comparative Example 3, in which a band of oxidized milk globulin protein is observed at 18 kDa. The electrophoresis images of the molecular weight markers are illustrated in (c) in FIG. 9.

### Implementation Example 2

In Implementation Example 2, after a predetermined voltage was applied to Sample Solution II at a low temperature overnight, 10 mM of AMS was added to Sample Solution II and Sample Solution II was incubated at 37°C for 1 hour. Sample Solution II was prepared the same as in Comparative Example 3.

### Reduction of Oxidized Milk Globulin

In Implementation Example 2, a predetermined voltage was applied to Sample Solution I overnight, at a low temperature, using a three-electrode voltage-applying cell (for example, voltage applier 10a in FIG. 1) and a potentiostat (for example, power supply 20 in FIG. 1). Among the three electrodes, a glassy carbon electrode was used for working electrode 1a and an Ag/AgCl electrode was used for reference electrode 2. The predetermined voltage applied to Sample Solution I was controlled by the potentiostat so that the potential of working electrode 1a relative to reference electrode 2 was equal to the reduction potential of the electron mediator.

### Modification of Reduced Proteins with Thiol Group Modifying Reagents

After Sample Solution II was allowed to stand for 16 hours, 10 mM of AMS was added to Sample Solution II and Sample Solution II was incubated at 37°C for 1 hour. Next, the incubated Sample Solution II and molecular weight markers were electrophoresed by a typical SDS-PAGE method. Next, the gel was stained after eletrophoresis. The electrophoresis images are illustrated in FIG. 9. In FIG. 9, (b) illustrates electrophoresis images for Implementation Example 2, in which a band of reduced milk globulin protein is observed at 19 kDa.

### (3) Reduction of Disulfide Bonds in Egg Albumin

Next, the reduction of disulfide bonds in digestion-resistant proteins was verified using egg albumin.

### Comparative Example 4

In Comparative Example 4, the process was the same as in Comparative Example 3, except that Sample Solution III was used instead of Sample Solution II.

### Preparation of Sample Solution III

Sample Solution III was prepared in the same manner as Sample Solution II, except that oxidized egg albumin was used instead of the target molecule, oxidized milk globulin.

### Reduction of Oxidized Egg Albumin

After Sample Solution III was prepared, Sample Solution III was allowed to stand for 16 hours at a low temperature (for example, the internal temperature of a refrigerator). No voltage was applied to Sample Solution III.

### Modification of Reduced Proteins with Thiol Group Modifying Reagents

After Sample Solution III was allowed to stand for 16 hours, 10 mM of AMS was added to Sample Solution III and Sample Solution III was incubated at 37°C for 1 hour. Next, the incubated Sample Solution III and molecular weight markers were electrophoresed by a typical SDS-PAGE method. Next, the gel was stained after eletrophoresis. The electrophoresis images are illustrated in FIG. 10. FIG. 10 illustrates electrophoresis images after SDS-PAGE in Comparative Example 4 and Implementation Example 3. In FIG. 10, (a) illustrates electrophoresis images for Comparative Example 4, in which a band of oxidized egg albumin protein is observed at 45 kDa. The electrophoresis images of the molecular weight markers are illustrated in (c) in FIG. 10.

### Implementation Example 3

In Implementation Example 3, the process was the same as in Implementation Example 2, except that Sample Solution III was used instead of Sample Solution II.

### Reduction of Oxidized Egg Albumin

In Implementation Example 3, the process was the same as in Implementation Example 2, except that Sample Solution III was used instead of Sample Solution II.

### Modification of Reduced Proteins with Thiol Group Modifying Reagents

After Sample Solution III was allowed to stand for 16 hours, 10 mM of AMS was added to Sample Solution III and Sample Solution III was incubated at 37°C for 1 hour. Next, the incubated Sample Solution II and molecular weight markers were electrophoresed by a typical SDS-PAGE method. Next, the gel was stained after eletrophoresis. The electrophoresis images are illustrated in FIG. 10. In FIG. 10, (b) illustrates electrophoresis images for Implementation Example 3, in which a band of reduced egg albumin protein is observed at 46 kDa.

### Observations

In Comparative Example 3 and Comparative Example 4, Sample Solution II and Sample Solution III were allowed to sit overnight without applying voltage, and then a thiol group modifying reagent was added and Sample Solution II and Sample Solution III were incubated. As a result, as illustrated in (a) in FIG. 9 and (a) in FIG. 10, the oxidized digestion-resistant proteins (more specifically, the disulfide bonds in the digestion-resistant proteins) in Sample Solution II and Sample Solution III were not reduced. On the other hand, in Implementation Example 2 and Implementation Example 3, after a predetermined voltage was applied to Sample Solution II and Sample Solution III, a thiol group modifying reagent was added and Sample Solution II and Sample Solution III were incubated. As a result, the mobility in SDS-PAGE shifted in the macromolecular direction, as illustrated in (b) in FIG. 9 and (b) in FIG. 10.

The above results suggest that when no voltage is applied to Sample Solution II and Sample Solution III, oxidized redox proteins in Sample Solution II and Sample Solution III are not reduced to reduced redox proteins. Stated differently, this suggests that if no voltage is applied to Sample Solution II and Sample Solution III, once a reduced redox protein in Sample Solution II and Sample Solution III is oxidized, it cannot be reduced again to a reduced redox protein. Thus, in Comparative Example 3 and Comparative Example 4, the disulfide bonds in the digestion-resistant proteins were not reduced because only enough disulfide bonds were cleaved to reduce the reduced redox proteins in Sample Solution II and Sample Solution III.

However, the results of Implementation Example 2 and Implementation Example 3 suggest that oxidized redox proteins in Sample Solution II and Sample Solution III are reduced to reduced redox proteins when voltage is applied to the sample solution. Stated differently, this suggests that when voltage is applied to Sample Solution II and Sample Solution III, once a reduced redox protein in Sample Solution II and Sample Solution III is oxidized, the oxidized redox protein is reduced again to a reduced redox protein by indirect electron transfer from the electrode. This suggests that Implementation Example 2 and Implementation Example 3 produce significant results even when a small amount of redox protein relative to the number of disulfide bonds is used because the application of a predetermined voltage to Sample Solution II and Sample Solution III repeatedly reduces the redox proteins in Sample Solution II and Sample Solution III thereby reducing the disulfide bonds in digestion-resistant proteins.

Although the method for cleaving disulfide bonds in proteins and the device for cleaving disulfide bonds in proteins according to the present disclosure have been described above based on embodiments, the present disclosure is not limited to these embodiments. Various modifications to the above embodiments that may be conceived by those skilled in the art, as well as embodiments resulting from arbitrary combinations of elements from different embodiments that do not depart from the essence of the present disclosure are included the present disclosure.

Although the above embodiments describe an example of efficient reduction of digestion-resistant proteins using electrical energy by controlling the application of voltage, the present disclosure is not limited to this example. For example, in Embodiment 2, sample 9b may be agitated to increase the electron transfer reactivity of the redox proteins immobilized on working electrode 1b and the digestion-resistant proteins in sample 9b. In other words, in addition to voltage application conditions, controller 30 of device 100b for cleaving disulfide bonds in proteins may derive agitation conditions such as agitation speed, agitation time, and agitation interval, and output a control signal related to voltage control and a control signal related to agitation control. In such cases, device 100b for cleaving disulfide bonds in proteins may include an agitator that agitates sample 9b in cell 4 of voltage applier 10b. The agitator may include an agitator blade that is attachable to and removable from lid 5, a motor that rotates the agitator blade, and a controller that controls the movement of the motor.

### [Industrial Applicability]

The present disclosure provides a method for cleaving disulfide bonds in proteins and a device that implements this method that can efficiently cleave disulfide bonds in proteins derived from various materials such as food, silk, or plants.

### [Reference Signs List]

- 1a, 1b: working electrode
- 2: reference electrode
- 3: counter electrode
- 4: cell
- 5: lid
- 6a, 6b, 6c: terminal
- 7a, 7b, 7c: lead
- 9a: sample solution
- 9b: sample
- 10a, 10b: voltage applier
- 11: substrate
- 12: conductive polymer
- 13: conductive particle
- 14: redox protein
- 15: redox enzyme
- 16: redox molecule
- 17: electron mediator
- 18: base electrode
- 20: power supply
- 21: obtainer
- 22: information processor
- 23: voltage controller
- 24: outputter
- 30: controller
- 31: obtainer
- 32: information processor
- 33: storage
- 34: outputter
- 100a, 100b: device for cleaving disulfide bonds in proteins

## Claims

1. A method for cleaving a disulfide bond in a protein, the method comprising:
cleaving a disulfide bond in a protein present in a reaction system by reduction via a reduced redox protein; and
reducing an oxidized redox protein produced by oxidation of the reduced redox protein in the cleaving to the reduced redox protein by donating an electron from an electrode connected to an external power supply outside the reaction system to the oxidized redox protein.

2. The method for cleaving a disulfide bond in a protein according to claim 1, wherein
in the reducing, an electron is donated from the electrode to a redox enzyme, and an electron is donated from the redox enzyme to the oxidized redox protein.

3. The method for cleaving a disulfide bond in a protein according to claim 1, wherein
in the reducing, an electron is donated from the electrode to a redox molecule, an electron is donated from the redox molecule to a redox enzyme, and an electron is donated from the redox enzyme to the oxidized redox protein.

4. The method for cleaving a disulfide bond in a protein according to claim 1, wherein
in the reducing, an electron is donated from the electrode to an electron mediator, an electron is donated from the electrode mediator to a redox molecule, an electron is donated from the redox molecule to a redox enzyme, and an electron is donated from the redox enzyme to the oxidized redox protein.

5. The method for cleaving a disulfide bond in a protein according to any one of claims 1 to 4, wherein
the protein present in the reaction system is a digestion-resistant protein.

6. The method for cleaving a disulfide bond in a protein according to claim 5, wherein
the digestion-resistant protein is at least one of prolamin, egg albumin, or β-lactoglobulin.

7. The method for cleaving a disulfide bond in a protein according to any one of claims 1 to 6, wherein
the redox protein is thioredoxin, glutathione, a protein with at least one thioredoxin-like domain, or a protein with at least one glutathione-like motif.

8. The method for cleaving a disulfide bond in a protein according to any one of claims 2 to 4, wherein
the redox enzyme is: (i) an enzyme that catalyzes reduction of oxidized thioredoxin, including a nicotinamide adenine dinucleotide phosphate (NADPH)-thioredoxin reductase or a ferredoxin-thioredoxin reductase; or (ii) an enzyme that catalyzes reduction of oxidized glutathione, including a glutathione reductase.

9. The method for cleaving a disulfide bond in a protein according to claim 3 or 4, wherein
the redox molecule is nicotinamide adenine dinucleotide phosphate or ferredoxin.

10. The method for cleaving a disulfide bond in a protein according to claim 4, wherein
the electron mediator is a compound with a bipyridine skeleton.

11. The method for cleaving a disulfide bond in a protein according to any one of claims 1 to 10, wherein
a reaction temperature in the reaction system is greater than or equal to 4 °C and less than 60 °C.

12. The method for cleaving a disulfide bond in a protein according to any one of claims 1 to 11, wherein
a voltage applied to the electrode by the external power supply is between -1.0 V and 0 V, inclusive.

13. A device for cleaving a disulfide bond in a protein, the device comprising:
an electrode for donating an electron to a redox protein that cleaves a disulfide bond in a protein by reduction by application of voltage;
a power supply that applies voltage to the electrode; and
a controller that controls application of voltage by the power supply.

14. The device for cleaving a disulfide bond in a protein according to claim 13, wherein
the redox protein is immobilized on the electrode.

15. The device for cleaving a disulfide bond in a protein according to claim 13, wherein
a redox enzyme that donates an electron to the redox protein is further immobilized on the electrode.

16. The device for cleaving a disulfide bond in a protein according to claim 15, wherein
a redox molecule that donates an electron to the redox enzyme is further immobilized on the electrode.

17. The device for cleaving a disulfide bond in a protein according to claim 16, wherein
an electron mediator that donates an electron to the redox molecule is further immobilized on the electrode.

18. The device for cleaving a disulfide bond in a protein according to claim 13 or 14, wherein
an electron mediator that mediates electron transfer between the electrode and the redox protein is immobilized on the electrode.
